# EUROPÄISCHE PATENTSCHRIFT

(11) **EP 3 257 543 B1**
(45) Veröffentlichungstag und Bekanntmachung des Hinweises auf die Patenterteilung: **16.09.2020**
(21) Anmeldenummer: 17001013.6
(22) Anmeldetag: 14.06.2017
(51) Int. Cl.: A61M 16/08, A61M 16/06, A61M 39/10, A61M 16/00

(54) **VORRICHTUNG FÜR DIE BEATMUNG, AUFWEISEND ANSCHLUSSSTUTZEN UND DREHHÜLSE**
DEVICE FOR MECHANICAL VENTILATION, HAVING CONNECTING STUB AND ROTARY SLEEVE
DISPOSITIF POUR LA RESPIRATION ARTIFICIELLE, COMPORTANT MANCHON DE RACCORDEMENT ET DOUILLE ROTATIVE

(30) Priorität: 16.06.2016 DE 102016007302
(43) Veröffentlichungstag der Anmeldung: 20.12.2017
(73) Patentinhaber: Löwenstein Medical Technology S.A., 2557 Luxembourg (LU)
(72) Erfinder: Eifler, Martin, 25348 Glückstadt (DE)
(74) Vertreter: Marx, Thomas

(56) Entgegenhaltungen:
- EP-A1- 2 465 564
- WO-A1-00/78381
- WO-A1-2006/133480
- WO-A1-2016/159889
- WO-A1-2017/021836
- CN-U- 204 745 354
- DE-A1- 3 339 988
- US-A- 3 731 684
- US-A1- 2012 055 471
- US-A1- 2015 151 071

## Beschreibung

### Gebiet der Erfindung

Die vorliegende Erfindung betrifft eine als Patienteninterface ausgebildete Beatmungsvorrichtung. Beatmungsvorrichtungen sind für Patienten von Bedeutung, die unter einer Störung der Atemluft- oder Sauerstoffzufuhr leiden, wobei dem Patienten über ein Patienteninterface welches als Vollgesichtsmaske, Nasenmaske oder Nasal Pillow-Maske ausgebildet sein kann, mit einer positiven Druckunterstützung Atemgas oder mit Sauerstoff angereicherte Luft zugeführt wird.

### Stand der Technik

Um das Patienteninterface mit dem Beatmungsschlauch zu verbinden, verwendet man in der Regel eine Drehhülse, die über eine lösbare Schnappverbindung mit einem Anschlussstutzen der Maske verbunden wird. Dies ermöglicht das Ableiten von Schlauchkräften über eine Drehfunktion und ermöglicht dem Patienten das leichte Trennen der Verbindung bei einer Therapieunterbrechung.

Die US 2015/0151071 A1 gehört zum Stand der Technik nach Artikel 54 (2) EPÜ und offenbart ein Nasalmaskensystem umfassend eine Kopfhalterungsanordnung, eine Winkelanschlussanordnung ("elbow assembly"), eine Luftversorgungsanordnung und eine Polsteranordnung. Eine Entkopplungsstruktur, beispielsweise ein Drehanschluss, ist zumindest teilweise mit einer Befestigungsregion ausgebildet, die die Winkelanschlussanordnung aufnimmt. Die Winkelanschlussanordnung weist ein Winkelstück ("elbow") auf und dient als Zwischenstück zwischen der Polsteranordnung und der Luftversorgungsanordnung. Das Winkelstück ist mit einer Drehhülse ("swivel cuff") befestigt oder angeschlossen, die eine Luftversorgungsrohranordnung aufnimmt und relativ zum Winkelstück frei drehbar gelagert ist. Die Drehhülse weist einen ringförmigen Rastring auf, der in eine ringförmige Nut des Winkelstücks zur Drehverbindung von Drehhülse und Winkelstück aufgenommen wird. Die Drehhülse weist eine auf ihrer Außenfläche gelagerte Kanalportion auf, die einen Anschlussstück der Luftversorgungsrohranordnung aufnimmt.

Die WO 00/78381 A1 gehört zum Stand der Technik nach Artikel 54 (2) EPÜ und offenbart einen an einer Nasen-Atemmaske und einer Stirnstützenvorrichtung befestigten Anschluss. Der Anschluss ist ebenfalls geeignet zur Verwendung mit einer Voll-(d.h., Nasen-Mund-)Atemmaske. Der Anschluss schließt ein Maskenende zum Verbinden in Fluidverbindung mit dem Inneren der Atemmaske und ein wesentlich senkrecht zum Maskenende angeordnetes Zufuhrleitungsende zum Verbinden in Fluidverbindung mit dem Auslass einer Zufuhrleitung für atembares Gas ein. Das Maskenende wird durch einen Haltering drehbar an die Maskenhülle gekoppelt. Der Anschluss schließt ebenfalls einen Gas-Washout-Abzugsdurchgang, einen Körperabschnitt, einen abnehmbaren Kappenabschnitt und einen abnehmbaren Drehverbinder ein. Die Leitung wird reibschlüssig nicht drehbar über ein Ende des Drehverbinders geschoben. Das andere Ende des Drehverbinders ist in drehbarem Schnappeingriff mit einem Abschnitt mit verringertem Durchmesser des Körperabschnitts. Der Abschnitt ist aus federnden Fingern geformt, um ein Biegen während des Schnappeingriffs mit dem Drehverbinder zu erlauben.

Die WO 2006/133480 A1 gehört zum Stand der Technik nach Artikel 54 (2) EPÜ und offenbart einen Winkelanschluss umfassend ein Winkelstück und eine Drehhülse, die mit dem Winkelstück drehbar gekoppelt ist. Der Winkel weist eine Einlassleitung mit einem Schlauchende umfassend mehrere elastisch verformbare Laschen, die so strukturiert sind, dass sie eine lösbare Schnappverbindung mit der Drehhülse ermöglichen. Jede Lasche umfasst einen sich radial erstreckenden Vorsprung, der in einer inneren Nut der Drehhülse einrastet.

Die WO 2017/021836 A1 gehört zum Stand der Technik nach Artikel 54 (3) EPÜ und offenbart einen Anschlussstück, das zur Kopplung mit einem Winkelstück vorgesehen ist. Ein erstes Ende des Anschlussstücks ist zur Kopplung mit dem Winkelstück vorgesehen. Mindestens die Außenfläche der Anschlussstückwand verjüngt sich derart über mindestens Teil seiner Länge, dass mindestens ein dem ersten Ende benachbarten Teil des Anschlussstücks weist eine Außendimension auf, die größer als eine Außendimension eines dem anderen Ende des Anschlussstücks benachbarten Teils des Anschlussstücks ist. Diese Verjüngung vereinfacht den Einsatz des anderen Endes des Anschlussstücks in einen Beatmungsschlauch. Die Innenfläche des Anschlussstücks weist eine Rippe auf, die eine Schnappverbindung mit einem oder mehreren Vorsprüngen am Winkelstück baut.

Die WO 2016/159889 A1 gehört zum Stand der Technik nach Artikel 54 (3) EPÜ und offenbart ein Griffteil zur Verwendung mit Beatmungsvorrichtungen. Ein zylindrisches Auslassende des Griffteils kann mit einer Maske bzw. einem Mundstück gekoppelt werden, während ein zylindrisches Einlassende mit einer Filtereinheit bzw. mit einem Schlauch gekoppelt wird.

Das Auslassende hat einen Standardaußendurchmesser von 22 mm. Der Griffteil umfasst ein bananenförmiges Rohr zwischen dem Einlassende und dem Auslassende. Der Griffteil weist eine dem Einlassende benachbarte, sich durch das Rohr erstreckende Öffnung auf. Der Griffteil weist einen Ring auf, der zwischen einer ersten Position, in der die Öffnung zur Umgebung geöffnet ist, und einer zweiten Position, in der die Öffnung geschlossen ist, drehbar gelagert ist. Das Rohr umfasst eine kegelstumpfförmige Region bzw. Wand sowie eine zylindrische Region bzw. Wand, die die kegelstumpfförmige Region mit dem zylindrischen Einlassende verbindet. Der Ring umfasst eine Hülse, die die Mehrheit der kegelstumpfförmigen und der zylindrischen Regionen des Rohres in drehbarer Rastverbindung umhüllt. Somit weist der Ring einen kegelstumpfförmigen Teil sowie einen zylindrischen Teil auf, die komplementär zu der kegelstumpfförmigen und der zylindrischen Regionen des Rohres sind. Das Rohr weist eine erste und eine zweite Aussparung auf, und der Ring weist eine flexible Lasche mit einem Anschlag auf, der zur Rastverbindung von Ring und Rohr in der ersten Position des Ringes in die erste Aussparung aufgenommen wird und in der zweiten Position des Ringes in der zweiten Aussparung aufgenommen wird.

### Kritik am Stand der Technik

Der Anschlussstutzen ist beispielsweise als Winkel mit einem Kugelgelenk ausgeführt. Die Schnappverbindung zwischen dem Anschlussstutzen und der Drehhülse wird in der Regel durch Schnapphaken im Anschlussstutzen realisiert. Der Anschlussstutzen ist hierfür geschlitzt ausgeführt, so dass sich federnde Elemente ergeben. Diese haben den Nachteil, dass sie leicht abbrechen können. Durch die Schlitzung ergibt sich außerdem eine geringe Überdeckung / Überlappung zwischen Anschlussstutzen und Drehhülse, so dass es zu erhöhten Leckagen kommt.

Ein weiteres Problem stellt das Abnehmen / Lösen des Schlauchs von der Drehhülse dar. Die Schlauchmuffe ist viel fester mit der Drehhülse verbunden als die Drehhülse mit dem Winkel. Zum Lösen der sehr fest sitzenden Verbindung der Schlauchmuffe des Beatmungsschlauches von der Drehhülse steht nur eine sehr kleine Halte- bzw. Bedienfläche zur Verfügung, was für gerade ältere bzw. motorisch eingeschränkte Patienten sehr schwer zu Händeln ist.

### Erfindung

Ausgehend vom Stand der Technik liegt der Erfindung die Aufgabe zu Grunde, eine Vorrichtung der einleitend genannten Art derart zu konstruieren, dass eine hohe Funktionalität, eine verbesserte Handhabung und eine hohe Zuverlässigkeit bereitgestellt wird.

Die Aufgabe wird erfindungsgemäß gelöst durch eine Vorrichtung nach Anspruch 1. Die Unteransprüche definieren bevorzugte Ausführungsbeispiele der Erfindung.

Die folgenden Figuren zeigen den Aufbau einer Schlauchanbindung an ein Pantienteninterface im Rahmen eines Ausführungsbeispiels der Erfindung.

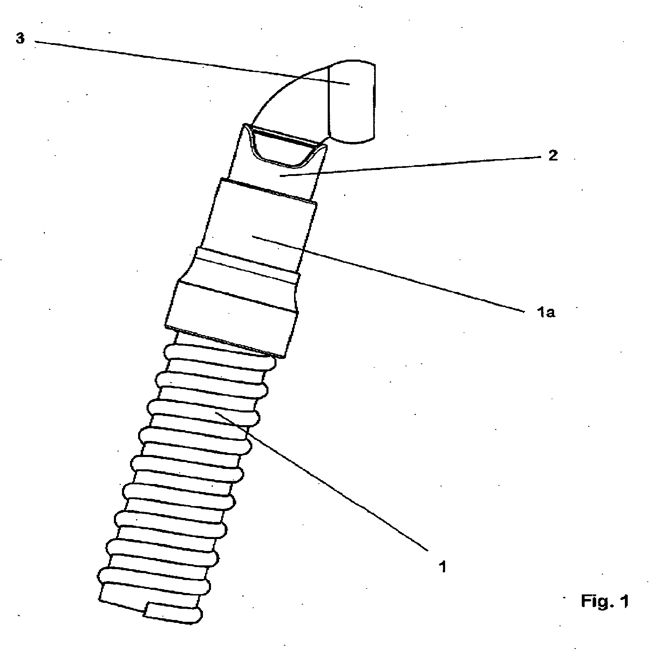

- Fig. 1:: Anschlussstutzen mit Drehhülse und Beatmungsschlauch

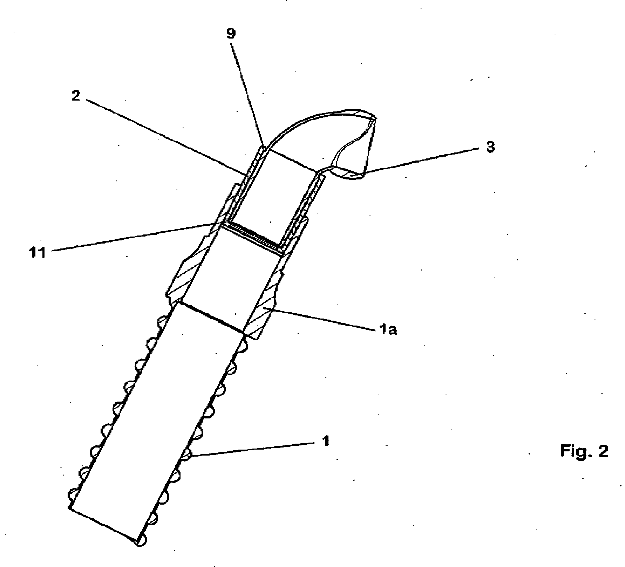

- Fig. 2:: Schnittdarstellung des Aufbaus

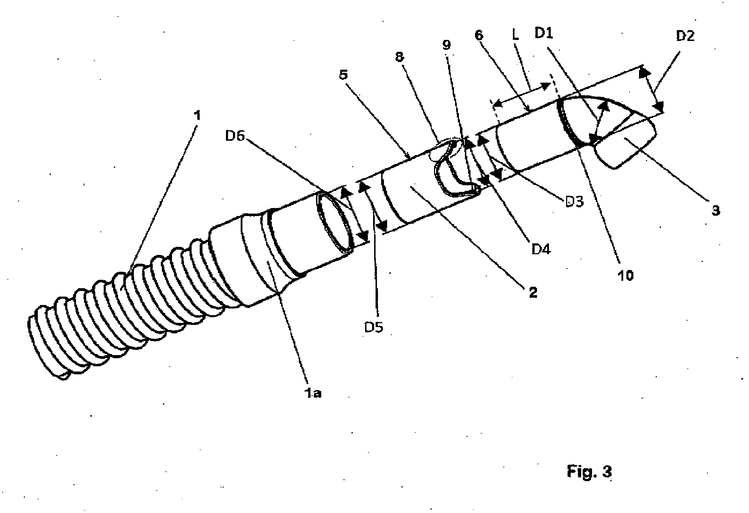

- Fig. 3:: Explosionsdarstellung des Aufbaus

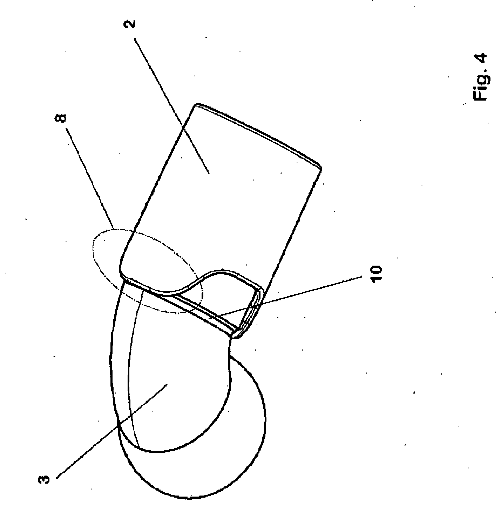

- Fig. 4:: Drehhülse mit Anschlussstutzen verbunden

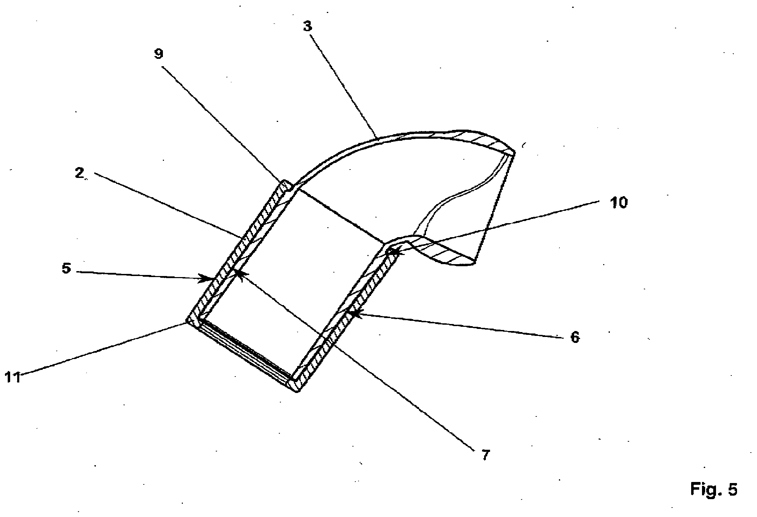

- Fig. 5:: Schnitt durch Drehhülse und Anschlussstutzen
- Fig. 6:: Drehhülse

Für die Beatmung wird der Schlauch (1) an dessen Enden sich eine Schlauchmuffe (1a) befinden, patientenseitig über eine Drehhülse (2) und einen Anschlussstutzen (6) mit dem (nicht dargestellten) Patienten Interface (4) verbunden. Die Schlauchmuffe (1a) des Beatmungsschlauchs (1) wird soweit über die konisch leicht dicker werdende Außenwand (5) der Drehhülse' (2) geschoben bis sie einen festen sitz aufweist. Dazu weist die Schlauchmuffe (1a) einen Innendurchmesser D6 aus, der größer ist als der Außendurchmesser D5 der Drehhülse (2). Die Drehhülse weist eine konisch leicht dicker werdende Außenwand (5) auf, weshalb sich der Außendurchmesser D5 der Drehhülse (2) langsam aufweitet bis zu dem, dem Anschlussstutzen zugewandten, Ende (8).

Die Drehhülse (2) weist beispielsweise neben der konischen Außenfläche (5) auch eine konische Innenfläche (7) auf. Diese ist korrespondierend mit dem Stutzen (6) des Winkelanschlusses (3) ausgebildet und ermöglicht durch ein geringes Spiel die leichtgängige Drehfunktion der Bauteile. Die Drehhülse (2) weist symmetrische Verlängerungen (8) der einstückigen (einteiligen) Hülle auf, die man als Laschen bezeichnen kann. Die Laschen (8) weisen einen innen angeordneten Hinterschnitt (9) oder Hakenabschnitte auf, der zur Verrastung am Stutzen (6) dient. Der Stutzen (6) weist für die Verrastung eine umlaufende Fase bzw. einen Absatz (10) an der Oberfläche auf, hinter dem sich der Stutzen leicht verjüngt, mit dem Außendurchmesser D1. Die Drehhülse (2) weist einen Innendurchmesser D4 auf, der kleiner als der Außendurchmesser (D3) des Anschlussstutzens (6) ist.

Die Laschen (8) greifen mit dem Hinterschnitt über den Absatz (10) und bilden so eine Schnappverbindung zwischen Drehhülse (2) und Stutzen (6). Der Anschlussstutzen (6) weist beispielsweise eine konische Oberfläche auf was dazu führt, dass der Außendurchmesser (D3) geringer ist als der Außendurchmesser D2.

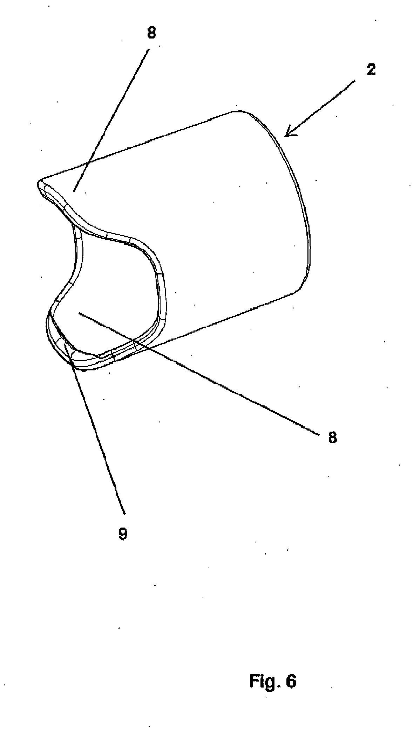

Ein axiales Verschieben zwischen der Drehhülse (2) und dem Stutzen wird durch einen Endanschlag (11) in der Drehhülse (2) vermieden, gegen den sich der stutzen (6) des Winkelanschlusses (3) maximal bewegen kann. Die Details der Schnappverbindung (Hinterschnitt 9 und Fase 10) sowie der Endanschlag (11) sind in der Schnittdarstellung zu erkennen. Der Hinterschnitt (9) an den Laschen (8) ist in der Detailansicht Fig. 6 der Drehhülse (2) dargestellt,

Soll die Verbindung zwischen Schlauch (1) und Maske (4) unterbrochen werden, reicht es an der Schlauchmuffe (1a) oder dem Schlauch (1) zu ziehen und die Schnappverbindung zwischen Stutzen (6) und Drehhülse (2) löst sich Die Schlauchmuffe (1a) sitzt aber immer noch sicher auf der Drehhülse (2). Soll aber, z.B. zur Reinigung, die Verbindung zwischen Schlauchmuffe (1a) und Drehhülse (2) gelöst werden, dienen die beiden verlängerten Laschen (8) zudem als Halte- bzw. Griffflächen. Zum Demontieren des Schlauchs fasst man die Drehhülse an den Laschen (8) an und sichert so gleichzeitig - durch den Druck auf die Laschen - die Schnappverbindung, wenn der Schlauch (1) durch leichtes Biegen der Schlauchmuffe (1a) von der Drehhülse (2) gezogen wird.

Durch den langen, glatten Überlappungsbereich zwischen der Drehhülse (2) und dem Stutzen kommt es zu wesentlich weniger Leckagen als bei den bekannten, geschlitzten Schnappverbindungen. Der Anschlussstutzen (6) wird dazu zumindest mit 30 % seiner Länge, bevorzugt mit zumindest 40 % seiner Länge und besonders bevorzugt mit zumindest 60 % seiner Länge in die Drehhülse (2) eingeführt.

### Bezugsziffern:

- 1: Beatmungsschlauch mit
- 1a: Schlauchmuffe
- 2: Drehhülse
- 3: Winkelanschluss mit Kugelgelenk
- 4: Beatmungsmaske (nicht dargestellt)
- 5: Außenfläche der Drehhülse (2)
- 6: Stutzen
- 7: Innenfläche der Drehhülse (2)
- 8: Lasche
- 9: Hinterschnitt
- 10: Fase, Absatz
- 11: Anschlag

## Patentansprüche

1. Vorrichtung für die Beatmung bestehend aus einem Anschlussstutzen (6), einer Drehhülse (2), einem Beatmungsschlauch (1) und einem Patienteninterface, wobei der Anschlussstutzen (6) konfiguriert ist zumindest teilweise in die Drehhülse (2) eingeführt und mit dieser verrastet zu sein, wobei der Anschlussstutzen (6) und die Drehhülse (2) konfiguriert sind, zwischen dem Beatmungsschlauch (1) und dem Patienteninterface angeordnet zu sein, wobei der Anschlussstutzen (6) einen Außendurchmesser (D3) aufweist, der geringer ist als ein Innendurchmesser (D4) der Drehhülse (2) und wobei die Drehhülse (2) an ihrer Innenseite Rastelemente (9) aufweist, die konfiguriert sind, in Rastelemente (10), die an einer Außenwand des Stutzens (6) ausgebildet sind, zu greifen, wobei die Drehhülse (2) konisch ist, weshalb sich der Außendurchmesser (D5) der Drehhülse (2) aufweitet bis zu dem Ende (8), das konfiguriert ist, dem Anschlussstutzen (6) zugewandt zu sein, wobei die Drehhülse (2) eine einstückige Außenwand (5) und symmetrische Verlängerungen (8) der einstückigen Außenwand (5) aufweist.

2. Vorrichtung nach Anspruch 1 **dadurch gekennzeichnet, dass** der Stutzen (6) dazu konfiguriert ist, mit zumindest 30 % seiner Länge in die Drehhülse (2) einzutauchen.

3. Vorrichtung nach einem der vorhergehenden Ansprüche **dadurch gekennzeichnet, dass** der Stutzen (6) dazu konfiguriert ist, mit zumindest 60 % seiner Länge in die Drehhülse (2) einzutauchen.

4. Vorrichtung nach zumindest einem der vorhergehenden Ansprüche **dadurch gekennzeichnet, dass** die Drehhülse (2) an ihrer Innenwand (7) einen Anschlag (11) für den Stutzen (6) aufweist.

5. Vorrichtung nach zumindest einem der vorhergehenden Ansprüche **dadurch gekennzeichnet, dass** der Beatmungsschlauch (1) eine Schlauchmuffe (1a) mit einem Innendurchmesser (D6) aufweist, der größer ist als ein Außendurchmesser (D5) der Drehhülse (2).

6. Vorrichtung nach zumindest einem der vorhergehenden Ansprüche, **dadurch gekennzeichnet, dass** die Innenwand (7) der Drehhülse (2) konisch ist.

7. Vorrichtung nach zumindest einem der vorhergehenden Ansprüche **dadurch gekennzeichnet, dass** die Verlängerungen (8) an ihrer Innenwand einen Hinterschnitt (9) oder Rastabschnitt aufweisen, der zur Verrastung mit dem Stutzen (6) dient.

8. Vorrichtung nach zumindest einem der vorhergehenden Ansprüche **dadurch gekennzeichnet, dass** der Stutzen (6) für die Verrastung mit der Drehhülse (2) einen Absatz (10) in der Außenwand aufweist.

9. Vorrichtung nach den Ansprüchen 7 und 8, **dadurch gekennzeichnet, dass** die Verlängerungen (8) konfiguriert sind, mit dem Hinterschnitt (9) über den Absatz (10) zu greifen und so eine Schnappverbindung zwischen Drehhülse (2) und Stutzen (6) zu bilden.

10. Vorrichtung nach zumindest einem der vorhergehenden Ansprüche **dadurch gekennzeichnet, dass** ein axiales Verschieben zwischen der Drehhülse (2) und dem Stutzen durch einen Endanschlag (11) in der Drehhülse (2), gegen den der Stutzen (6) stößt, vermieden wird.

## Claims

1. A device for mechanical ventilation, consisting of a connecting piece (6), a rotary sleeve (2), a breathing tube (1) and a patient interface, wherein the connecting piece (6) is configured to be at least partially inserted into the rotary sleeve (2) and locked with this, wherein the connecting piece (6) and the rotary sleeve (2) are configured to be arranged between the breathing tube (1) and the patient interface, wherein the connecting piece (6) has an external diameter (D3) which is smaller than an internal diameter (D4) of the rotary sleeve (2), and wherein the rotary sleeve (2) has on its inner side locking elements (9) which are configured to engage in locking elements (10) which are formed on an outer wall of the connector (6), wherein the rotary sleeve (2) is conical, which is why the external diameter (D5) of the rotary sleeve (2) flares up to the end (8) which is configured to face the connecting piece (6), wherein the rotary sleeve (2) has an integral outer wall (5) and symmetrical extensions (8) of the integral outer wall (5).

2. The device according to Claim 1, **characterized in that** the connector (6) is configured to be inserted with at least 30% of its length into the rotary sleeve (2).

3. The device according to any one of the preceding claims, **characterized in that** the connector (6) is configured to be inserted with at least 60% of its length into the rotary sleeve (2) .

4. The device according to at least one of the preceding claims, **characterized in that** the rotary sleeve (2) has on its inner wall (7) a stop (11) for the connector (6).

5. The device according to at least one of the preceding claims, **characterized in that** the breathing tube (1) has a tube fitting (1a) having an internal diameter (D6) which is greater than an external diameter (D5) of the rotary sleeve (2).

6. The device according to at least one of the preceding claims, **characterized in that** the inner wall (7) of the rotary sleeve (2) is conical.

7. The device according to at least one of the preceding claims, **characterized in that** the extensions (8) on its inner wall have an undercut (9) or latching portion which is used for locking with the connector (6).

8. The device according to at least one of the preceding claims, **characterized in that** the connector (6) has a shoulder (10) in the outer wall for locking with the rotary sleeve (2).

9. The device according to Claims 7 and 8, **characterized in that** the extensions (8) are configured to engage with the undercut (9) via the shoulder (10) and to thus form a snap-in connection between the rotary sleeve (2) and the connector (6).

10. The device according to at least one of the preceding claims, **characterized in that** an axial displacement between the rotary sleeve (2) and the connector is avoided by an end stop (11) in the rotary sleeve (2), against which the connector (6) abuts.

## Revendications

1. Dispositif pour la respiration artificielle, constitué d'un manchon de raccordement (6), d'une douille rotative (2), d'un tube respiratoire (1) et d'une interface patient, dans lequel le manchon de raccordement (6) est configuré pour être introduit au moins partiellement dans la douille rotative (2) et encliqueté dans celle-ci, dans lequel le manchon de raccordement (6) et la douille rotative (2) sont configurés pour être disposés entre le tube respiratoire (1) et l'interface patient, dans lequel le manchon de raccordement (6) présente un diamètre extérieur (D3) inférieur à un diamètre intérieur (D4) de la douille rotative (2) et dans lequel la douille rotative (2) présente des éléments d'encliquetage (9) sur son côté intérieur, lesquels sont configurés pour s'engager dans des éléments d'encliquetage (10) formés sur une paroi extérieure du manchon (6), dans lequel la douille rotative (2) est conique, moyennant quoi le diamètre extérieur (D5) de la douille rotative (2) s'élargit jusqu'à l'extrémité (8) configurée pour être tournée vers le manchon de raccordement (6), dans lequel la douille rotative (2) présente une paroi extérieure monobloc (5) et des prolongements symétriques (8) de la paroi extérieure monobloc (5).

2. Dispositif selon la revendication 1, **caractérisé en ce que** le manchon (6) est configuré pour plonger dans la douille rotative (2) sur au moins 30% de sa longueur.

3. Dispositif selon l'une des revendications précédentes, **caractérisé en ce que** le manchon (6) est configuré pour plonger dans la douille rotative (2) sur au moins 60% de sa longueur.

4. Dispositif selon l'une au moins des revendications précédentes, **caractérisé en ce que** sur sa paroi intérieure (7), la douille rotative (2) présente une butée (11) pour le manchon (6).

5. Dispositif selon l'une au moins des revendications précédentes, **caractérisé en ce que** le tube respiratoire (1) présente un embout de tube (1a) avec un diamètre intérieur (D6) supérieur au diamètre extérieur (D5) de la douille rotative (2).

6. Dispositif selon l'une au moins des revendications précédentes, **caractérisé en ce que** la paroi intérieure (7) de la douille rotative (2) est conique.

7. Dispositif selon l'une au moins des revendications précédentes, **caractérisé en ce que** sur leur paroi intérieure, les prolongements (8) présentent une contre-dépouille (9) ou une section d'encliquetage servant à l'encliquetage avec le manchon (6).

8. Dispositif selon l'une au moins des revendications précédentes, **caractérisé en ce que** le manchon (6) présente un gradin (10) pour l'encliquetage avec la douille rotative (2).

9. Dispositif selon les revendications 7 et 8, **caractérisé en ce que** les prolongements (8) sont configurés pour s'engager avec la contre-dépouille (9) sur le gradin (10) et pour ainsi former une liaison à enclenchement entre la douille rotative (2) et le manchon (6).

10. Dispositif selon l'une au moins des revendications précédentes, **caractérisé en ce qu'**un déplacement axial entre la douille rotative (2) et le manchon est empêché par une butée finale (11) dans la douille rotative (2), contre laquelle s'applique le manchon (6).
